# EUROPEAN PATENT SPECIFICATION

(11) **EP 1 471 138 B1**
(45) Date of publication and mention of the grant of the patent: **24.08.2011**
(21) Application number: 04252263.1
(22) Date of filing: 16.04.2004
(51) Int. Cl.: C12M 1/00, C12M 1/12, C12M 3/00, A61L 2/04, A61L 2/24, G01N 35/00, G21F 7/005

(54) **Aseptic system and method for using the same**
Aseptisches System und Verfahren zu dessen Verwendung
Système aseptique et méthode d'utilisation

(30) Priority: 25.04.2003 JP 2003122465
(43) Date of publication of application: 27.10.2004
(73) Proprietor: SHIBUYA KOGYO CO., LTD, Kanazawa-Shi Ishikawa-Ken (JP)
(72) Inventor: Kokubo, Mamoru - Koh, Kanazawa-Shi Ishikawa-Ken (JP); Ueda, Hirosho - Koh, Kanazawa-Shi Ishikawa-Ken (JP)
(74) Representative: Naylor, Matthew John

(56) References cited:
- WO-A-01/91810
- WO-A-91/01365
- US-A- 5 421 626
- US-A- 5 525 512
- US-A- 5 863 498
- US-A1- 2003 232 429
- US-A1- 2004 002 415
- PATENT ABSTRACTS OF JAPAN vol. 199, no. 813, 30 November 1998 (1998-11-30) & JP 10 216207 A (UDONO IKI KK), 18 August 1998 (1998-08-18)

## Description

### Field of the Invention

The present invention relates to an aseptic system and a method for using the same, more specifically an aseptic system comprising an isolator which is isolated from the external atmosphere and maintained in an aseptic condition in the inside, and a method for using the same.

### Description of the Prior Art

Today, various aseptic systems as below are used in order to conduct various operations such as incubating of a microorganism in an aseptic condition associated with the asepticism test for medical supplies and incubating of cells taken from a human body or an animal.

First, there is known an aseptic system comprising two or more serially connected aseptic boxes or aseptic operation boxes (Patent Document 1: Japanese Utility Model Pre-Grant Publication No. 56-47741), and cells can be incubated in an aseptic condition by using each of these aseptic boxes or aseptic operation boxes as an incubator or a clean booth.

Second, there is known an incubate equipment comprising an aseptic testing bench and an incubator connected through an opening-and-closing door provided on the incubator (Patent Document 2: Japanese Utility Model Granted Publication No. 62-7110). Operations such as addition of an incubate solution to the cells in an incubation container are conducted within the aseptic testing bench and then the incubator is moved into the incubator, thereby the cells can be incubated in an aseptic condition.

First, in the case of the construction of the above-mentioned Patent Document 1, although there is a scalability by connecting two or more aseptic boxes or aseptic operation boxes, each of the aseptic boxes or aseptic operation boxes can be only serially connected and therefore the operator should move to the respective aseptic box or aseptic operation box for conducting each operation and the operation is inefficient.

Further, in the case of the construction of the above-mentioned Patent Document 2, although an air curtain prevents that harmful substances from flowing out to the exterior, when an incubation container after the completion of incubation is taken out of the aseptic testing bench to the exterior, microorganisms may adhere to the surface of the incubation container or present among the cells in the incubation container, which causes a risk that these microorganisms may flow out to the exterior with an incubation container.

### Summary of the Invention

In view of the above, the present invention provides an aseptic system according to claim 1. The aseptic system has a high scalability and operation efficiency and prevents a harmful microorganism and the like from flowing out to the exterior.

Preferably, the isolator is formed in a polygonal shape with five or more side walls at least four of which have communicating openings to the outside and each of the communicating openings is provided with an opening-and-closing door respectively.

In another aspect there is provided a method for using an aseptic system, according to claim 6.

According to one embodiment, the isolator has five or more side walls and four or more communicating openings and accordingly various kinds of equipments required for operation in an aseptic condition can be connected, thereby a highly scalable aseptic system can be obtained. In addition, the isolator is located at the middle of these equipments, the operator does not need move around for conducting operation in an aseptic condition as in the invention of the above-mentioned Patent Document 1, and can conduct efficient operation.

Since the isolator is provided with surface sterilizing means and heating and sterilizing means, harmful microorganisms can be prevented from flowing out to the exterior of the aseptic system.

Furthermore, according to yet another embodiment, an incubation container can be taken out to the exterior from the isolator, while annihilating not only microorganisms adhering to the surface of the incubation container but also microorganisms in the incubation container by heating depending on the state of incubation in case that the incubate has any abnormalities, and it can be prevent that harmful microorganisms will flow out to the exterior from the aseptic system.

### Brief Description of the Drawings

FIG. 1 shows a plane view of the incubate equipment according to the first embodiment of the present invention;
FIG. 2 shows a plane view of the incubate equipment according to the second embodiment of the present invention; and
FIG. 3 shows a plane view of the incubate equipment 1 according to the third embodiment of the present invention.

### Detailed Description of the Embodiments

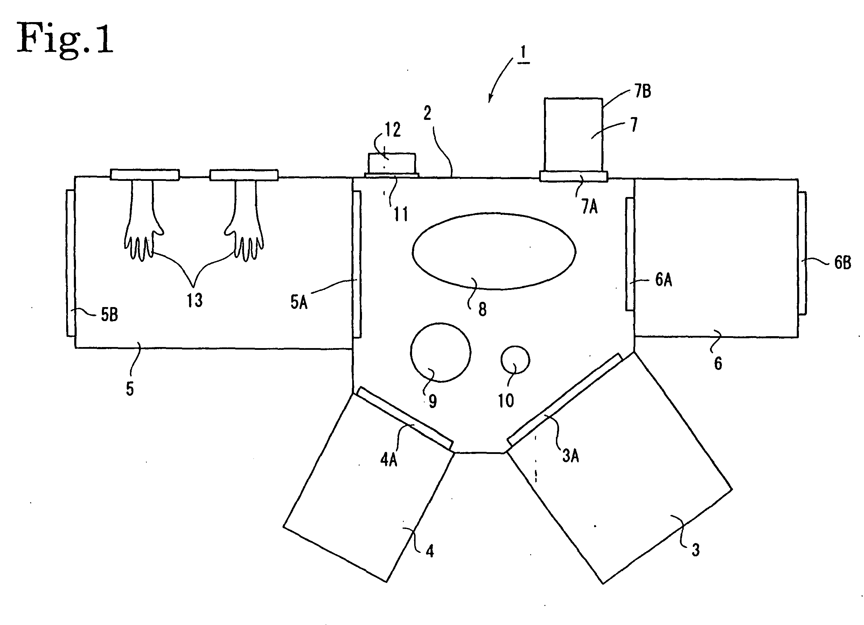

Referring to the appended drawings showing the embodiments of the invention, FIG. 1 shows an incubate equipment 1 which functions as an aseptic system for incubating cells from a human body or an animal. Cells are taken from a human body or an animal in a container (not illustrated) and then extracted from the body fluid and dispensed into an incubation container (not illustrated) and incubated in the incubate equipment 1.

As shown in FIG. 1, the incubate equipment 1 comprises an isolator 2 formed in a polygonal shape on the plane with six side walls and the isolator 2 is enclosed by the side walls, top panel and floor panel, and is isolated from the external atmosphere.

An incubator 3 for incubating cells is connected to the lower right side wall among these side walls in the drawing through a connection opening and a refrigerator 4 which refrigerates the incubate solution is connected to the lower left side wall through a connection opening.

Moreover, surface sterilizing means 5 to sterilize an incubation container etc. with a sterilization gas is connected to the side wall on the left-hand side in the drawing through a connection opening, and sterilizing means 6 is connected to the right-hand side wall through a connection opening which conducts sterilization with a high temperature and high pressure steam.

Furthermore, RTP (Rapid Transfer Port) 7 which accommodates materials which have become unnecessary is connected to the upper side wall through a connection opening, and this RTP 7 is provided to the isolator 2 so that it may be detached from the isolator 2 as described below.

Each connection opening which connects the isolator 2 with each means of the above-mentioned incubator 3, refrigerator 4, surface sterilizing means 5, heating and sterilizing means 6 and RTP 7 is provided with an opening-and-closing doors 3A, 4A, 5A, 6A and 7A so that the doors can be freely opened and closed and that if the opening-and-closing door is closed each of the above-mentioned means and the isolator 2 will be isolated in airtight.

There are also provided inside the isolator 2 a half suit 8 which the operator wears inside the isolator 2 and conducts necessary operation, a centrifugal separator 9 by which the above-mentioned body fluid is centrifuged, and observation means 10 for observing the incubated cells, and also reading means 12 for reading the bar code stuck on the container through the acrylics board 11 which is formed in the top end side wall of the isolator 2 in the drawing.

The isolator 2 is equipped with an aseptic air supply equipment which is not illustrated, and the inside of the isolator 1 is maintained at a predetermined positive pressure with aseptic air supplied from the aseptic air supply equipment.

The above-mentioned half suit 8 is positioned mostly at the center of the isolator 2 and the side walls of the isolator 2 are positioned so that the operational range in which the operator wearing the half suit 8 can conduct operations encompasses each of the above-mentioned means beyond the opening-and-closing doors 3A, 4A, 5A, 6A, and 7A as well.

Any conventionally known centrifugal separator may be used as a centrifugal separator 9, and the body fluid in the above-mentioned container is centrifuged into supernatant liquid and precipitation liquid.

As for the observation means 10, any conventionally known electron microscope may be used and the observation means 10 displays the observed images on a monitor (not illustrated) and simultaneously stores the images in a memory means (not illustrated).

Furthermore, the reading means 12 reads the bar code stuck on the container and transmits the contents of this bar code to the above-mentioned memory means. And the memory means memorizes the object from which the cells are taken and the time and/or date of such operation for every container.

In addition, the isolator 2 is equipped with a tray on which materials required for incubating cells are laid including a pipet for extracting a required ingredient from the container and these materials are carried into the isolator 2, after being sterilized by the above-mentioned surface sterilizing means 5 and/or the heating and sterilizing means 6.

Next, the incubator 3 can accommodate two or more incubation containers, and may be equipped with means (not illustrated) for adjusting the temperature and the humidity which maintains the inside of the incubator 3 at a necessary temperature and necessary humidity, and enables incubating of cells in the optimal environment.

The refrigerator 4 accommodates an incubate solution tank (not illustrated) which stores an incubate solution to be used and the inside of the refrigerator 4 is set at a predetermined temperature in order to prevent the incubate solution from being degraded by chemical change.

The surface sterilizing means 5 is provided with gloves 13 for conducting operation inside the surface sterilizing means 5 and a door 5B for carrying in and out a body fluid containing container and other materials from the exterior of incubate equipment 1 on the opposite side to the isolator 2.

The surface sterilizing means 5 is also provided with sterilization gas supply means (not illustrated) to supply sterilization gas such as hydrogen peroxide vapor into the surface sterilizing means 5 and the surface of an incubation container and materials are sterilized by filling the surface sterilizing means 5 with a sterilization gas.

The heating and sterilizing means 6 is an autoclave (high-pressure steam sterilization machine) and the heating and sterilizing means 6 is provided with a door 6B for carrying in and out an incubate solution and other materials from the exterior of incubate equipment 1 on the opposite side to the isolator 2.

This heating and sterilizing means 6 is adapted to heat and sterilize the incubation container and other materials and can be also used to annihilate the cells and microorganisms in the incubation container after the incubating has been completed.

Since the above RTP 7 is already disclosed by Japanese Patent Pre-Grant Publication No. 6-193323 etc., detailed explanation is omitted, a communicating opening to this RTP 7 and an opening-and-closing door 7A are respectively and separably provided to the side of the isolator 2 and to the side of the case 7B.

And the materials which have been used and needed no more within the isolator 2 are accommodated in the case 7B and the RTP 7 is separated from the isolator 2 at a predetermined time, and internal materials thereof are disposed.

As mentioned above, since the isolator of this example has six side walls can be connected to each means of the above-mentioned incubator 3, refrigerator 4, surface sterilizing means 5, heating and sterilizing means 6 and RTP 7 through five communicating opening, it is highly scalable.

And since each of the operating means such as the incubator 3 is connected within the operating range of the operator putting on the half suit 8, the operator can conduct necessary operations for incubating cells without moving around. Furthermore, since the side wall used in front of the operator putting on the half suit 8 is formed so that it tilts toward the half suit 8 and different operation means are arranged thereon respectively, the operator can work efficiently even the operator cannot turn back due to the wearing of the half suit 8.

Furthermore, since the centrifugal separator 9 is provided in the isolator 2, incubating and separation of the cells can be conducted in an aseptic condition, and invasion of microorganisms into the incubation container can be prevented when the separated cells are transferred to the incubator 3.

In the following, the usage of the aseptic system in this example is described. First, the inside of the isolator 2 is sterilized with sterilization gas such as hydrogen peroxide vapor from the sterilization gas supply means (not illustrated) before use. The opening-and-closing doors 3A and 3B are opened wide in this step so that the inside of the incubator 3 and the refrigerator 4 may be sterilized simultaneously.

Moreover, heatable materials among the materials to be carried into the inside of the isolator 2 for incubating and experiment such as incubation containers and experiment instruments are carried into the heating and sterilizing means 6 and those materials not heatable into the surface sterilizing means 5 on this occasion respectively through the doors 5B and 6B for carrying in and out and sterilized, and the surface of the container containing a body fluid of human or animal from which the cells to be incubated in the isolator 2 are taken out is simultaneously sterilized with the surface sterilizing means 6.

When the above sterilization operation is completed, the operator wears the half suit 8 provided in the isolator 2, carries in the sterilized materials and the container containing a body fluid into the isolator 2, and holds up the container to reading means 12 so that the data included in the bar code attached to the container may be read by the reading means 12.

The operator then sets this container to the centrifugal separator 9 and conducts centrifugal separation of the body fluid contained in a container. After that, the operator extracts the precipitation liquid containing the cells contained and dispenses the extract into two or more incubation containers.

Next, the operator opens the opening-and-closing door 4A of the refrigerator 4, pours a predetermined quantity of incubate solution from the incubate solution tank to an incubation container, and the operator further opens the opening-and-closing door 3A of the incubator 3, and places the incubation container in the incubator 3 to effect incubating.

After a predetermined period passes, the operator wears the half suit 8 and takes out each incubation container from the incubator 3, and check the existence of harmful bacteria and/or the form of the cell using the observation means 10.

Consequently, when judging that the cell in this incubation container is unnecessary for the reason, for example, that harmful bacteria and or microorganisms are present among the cells, the operator opens the opening-and-closing door 6A of the heating and sterilizing means 6, and places the incubation container in the heating and sterilizing means 6.

After the opening-and-closing door 6A is closed, the incubation container is sterilized by the heating and sterilizing means 6 by high-pressure steam sterilization and the cells in the incubation container will be sterilized along with microorganisms. And this incubation container is taken out from the taking-out door 9B to the exterior, and the extinct cells and microorganisms are discarded.

Thus, the microorganisms not only on the surface of the incubation container but in the incubation container are annihilated, these microorganisms can be prevented from flowing out to the exterior of the incubate equipment.

On the other hand, if the operator observes and judges that the cells in the incubation container are incubated normally, the operator returns the incubation container to the incubator 3 again, and will further continue incubating for a predetermined period.

And when this predetermined period passes and the cells are fully incubated, the operator takes out the incubation container from the incubator 3, opens the opening-and-closing door 5A of the surface sterilizing means 5 and places this incubation container in the surface sterilizing means 5. And the surface of the incubation container is sterilized with sterilization gas, and after that this incubation container is taken out from taking-out door 5B to the exterior of the incubate equipment 1.

Thus, because only the surface of the incubation container is sterilized, even if a harmful microorganism should adhere to the surface of an incubation container for some reason, for example, in the isolator 2, this can be sterilized while the cells in the incubation container will not be sterilized. Accordingly, the cells can be taken out to the exterior of the incubate equipment 1 in the safe state.

When the above incubation work is completed, after sterilizing used experiment instrument and the other materials with the surface sterilizing means 5 or the heating and sterilizing means 6 and carrying them out, the inside of the isolator 2 and incubator 3 and refrigerator 4 are sterilizes with sterilization gas like as-before use, and all the operations are completed.

When another sample is subjected to incubating, sterilization operation is performed before use and the inside of the isolator 2 is sterilized for every sample as mentioned above, cross contamination between samples will be prevented.

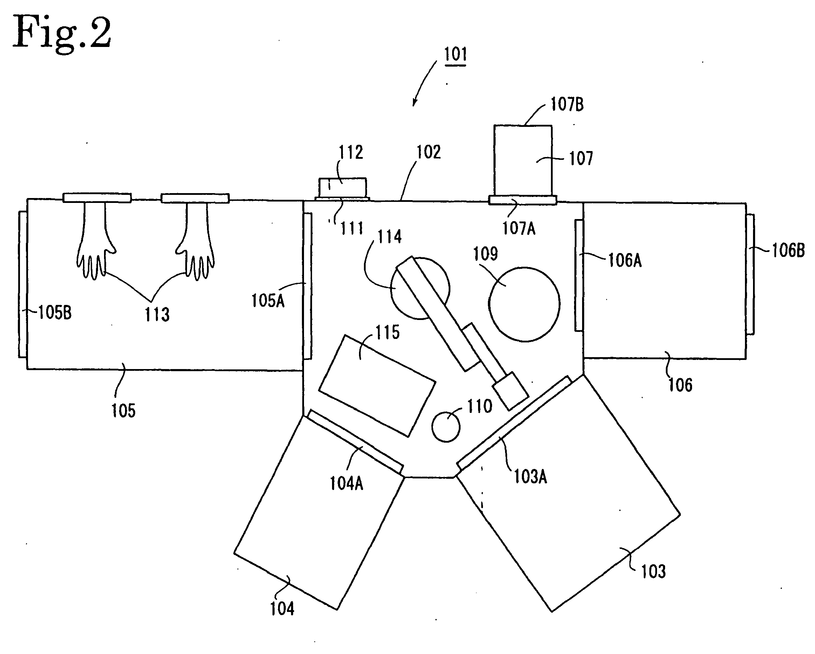

FIG. 2 shows the second example. In this example, a robot 114 is installed in the isolator 102 in places of the above-mentioned half suit 8, and what is common in the above first example in FIG. 2 is designated by a reference number which is a corresponding reference number in the first example added with 100.

While the robot 114 which operates with the control device (not illustrated) is installed in the isolator 102, isolator 102 is also provided with a head mount 115 for installing a head of the robot 114 which is exchanged according to the operation of the robot 114, and, for example, a gripper conveying an incubation container and a dispenser head for pouring the incubate solution into the incubation container are mounted.

Similarly as in the first example, the positions of the side walls of the isolator 102 are set so that the operational range of the robot 114 encompasses each of the above-mentioned means over the above-mentioned sealing doors 103A, 104A, 105A, 106A and 107A and that the robot can operate within these means. The opening-and-closing doors 103A, 104A, 105A, 106A, and 107A of this example are automatically opened and closed by the above-mentioned control device.

Furthermore, the images of the observation means 110 are displayed on the monitor provided outside of the incubate equipment 101, and the operator can watch this monitor to judge the state of the cells in the incubation container, and can give directions about processing of this incubation container to the control device.

In this example, the incubating of the cells is performed in the same way as in the first example described above, and accordingly, details about the operation of the robot 114 are omitted.

Thus, in the second example in which a robot 114 is provided within the isolator 102 in place of the half suit 8, the robot 114 is located approximately at the center of the isolator 102 and each of the side walls formed towards the robot 114 is provided with different operational means respectively so that each operational means is arranged radially from the robot 114. Accordingly, the operational range of the robot 114 is made small, which enables the robot to work effectively, and the reach of the robot 114 to be shortened, thereby the robot 114 can be made small to construct the whole equipment in a compact way.

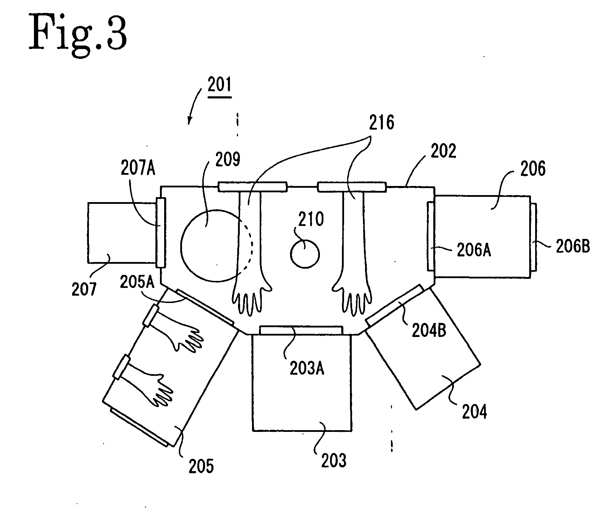

FIG. 3 shows the third example. This example has a construction wherein a glove(s) 216 is provided in the isolator 202, instead of providing a half suit 8 in the first example or a robot 114 in the second example. What is common in the first example of the above in FIG. 3 is designated by a reference number which is a corresponding reference number in the first example added with 200.

The isolator 202 also comprises six side walls in this example, and the upper one as illustrated here is constituted of a transparent acrylics board so that the operator can see the inside of the isolator.

Moreover, RTP 207 and the heating and sterilizing means 206 are connected to the right and left side walls in the drawing, and the surface sterilizing means 205, the incubator 203, and the refrigerator 204 are respectively connected to the lower side wall as illustrated sequentially from the left.

Furthermore, the positions of the side walls of the isolator 202 are set so that the operational range of the operator wearing the glove(s) 216 encompasses each of the above-mentioned means over the above-mentioned sealing doors 203A, 204A, 205A, 206A and 207A and that the operator can conduct operation within these means.

Therefore, as in the first example, the isolator 202 of this example are connected with operation means such as an incubator 203 within the operational range of the operator wearing the glove(s) 216 and therefore the operator can conduct operation required for incubating of the cells without moving around, and since the side wall in front of was the operator is tilted toward the glove(s) 216, the operator can work efficiently.

Although the three above-mentioned examples are concerned with incubating of the cells, the aseptic system which uses the above-mentioned isolator is applicable to preparation of medicine and the other examinations performed in an aseptic condition, and what equipment is connected to the connection opening of the above-mentioned isolator can be suitably varied depending on the purpose.

Moreover, although a single half suit 8, a robot 114 and a glove 216 were installed in the isolator 2, 102 and 202 respectively in each of the above-mentioned examples, two or more of them can be provided in the isolator 2, 102 and 202. For example, if a robot 114 and a glove 216 are installed in the isolator 2, the operator is able to do difficult work for the robot 114.

## Claims

1. An aseptic system (1) comprising an isolator (2) which is isolated from the external atmosphere and maintained in an aseptic condition in the inside, surface sterilizing means (5) to sterilize the surface of an object to be sterilized, the surface sterilizing means (5) having a door (5B) for carrying materials in from the exterior of the system (1) and for carrying materials out to the exterior of the system (1), the aseptic system further comprising heating and sterilizing means (6) to heat and sterilize an object to be sterilized, the heating and sterilizing means (6) having a door (6B) for carrying materials in from the exterior of the system (1) and for carrying materials out to the exterior of the system (1),
**characterised in that** the system further comprises an incubator (3) and wherein at least three of the isolator side walls have communicating openings to the surface sterilizing means (5), to the heating and sterilizing means (6) and to the incubator (3) respectively, and each of the communicating openings is provided with an opening-and-closing door (5A, 6A, 3A) respectively for opening and closing the communicating openings.

2. The aseptic system according to claim 1, wherein the isolator is formed in a polygonal shape with five or more side walls and at least a further one of said five or more side walls has a communicating opening to the outside and the communicating opening is provided with an opening-and-closing door (4A, 7A) for opening and closing the communicating opening.

3. The aseptic system according to claim 1 or claim 2, wherein there is provided at least either one of a half suit (8), a glove (216) and a robot (114) in the isolator and the operational range of the half suit, glove and robot reaches the range beyond the above-mentioned communicating opening.

4. The aseptic system according to any one of claims 1 to 3, wherein the incubating of cells is conducted in the incubator.

5. The aseptic system according to any one of claims 1 to 4, wherein the isolator is provided with a centrifugal separator (9) and a body fluid obtained from human body or animal is centrifuged therewith.

6. A method for using an aseptic system (1) comprising an isolator (2) which is isolated from the external atmosphere and maintained in an aseptic condition in the inside, surface sterilizing means (5) to sterilize the surface of an incubation container and heating and sterilizing means (6) to heat and sterilize an incubation container, wherein when the incubation container is taken out to the exterior from the isolator (2), in the case that the incubating in the incubation container has proceeded normally, the surface of the incubation container is sterilized by the surface sterilizing means (5), and in the case that the incubating in the incubation container has not proceeded normally, the incubation container is heated and sterilized by the heating and sterilizing means (6).

## Patentansprüche

1. Aseptisches System (1), umfassend einen Isolator (2), der von der äußeren Atmosphäre isoliert ist und in einem aseptischen Zustand im Inneren aufbewahrt wird, eine Oberflächensterilisierungsvorrichtung (5) zum Sterilisieren der Oberfläche eines zu sterilisierenden Gegenstands, wobei die Oberflächensterilisierungsvorrichtung (5) eine Tür (5B) zum Hineinbringen von Materialien von außerhalb des Systems (1) und zum Hinaustragen von Materialien aus dem System (1) heraus aufweist, wobei das aseptische System weiters eine Erhitzungs- und Sterilisierungsvorrichtung (6) zum Erhitzen und Sterilisieren eines zu sterilisierenden Gegenstands umfasst, wobei die Erhitzungs- und Sterilisierungsvorrichtung eine Tür (6B) zum Hineinbringen von Materialien von außerhalb des Systems (1) und zum Hinaustragen von Materialien aus dem System (1) heraus aufweist,
**dadurch gekennzeichnet, dass** das System weiters einen Inkubator (3) umfasst, und worin zumindest drei der Seitenwände des Isolators kommunizierende Öffnungen zur Oberflächensterilisierungsvorrichtung (5) hin, zur Erhitzungs- und Sterilisierungsvorrichtung (6) hin bzw. zum Inkubator (3) hin aufweisen und jede der kommunizierenden Öffnungen mit einer sich öffnenden und schließenden Tür (5A, 6A bzw. 3A) zum Öffnen und Schließen der kommunizierenden Öffnungen ausgestattet ist.

2. Aseptisches System nach Anspruch 1, worin der Isolator in polygonaler Form mit fünf oder mehreren Seitenwänden ausgebildet ist, zumindest eine weitere der fünf oder mehr Seitenwände eine kommunizierende Öffnung nach außen aufweist und die kommunizierende Öffnung mit einer sich öffnenden und schließenden Tür (4A, 7A) zum Öffnen und Schließen der kommunizierenden Öffnung ausgestattet ist.

3. Aseptisches System nach Anspruch 1 oder Anspruch 2, worin zumindest eines von einem Halbanzug (8), einem Handschuh (216) oder einem Roboter (114) im Isolator bereitgestellt ist und der Betriebsbereich des Halbanzugs, des Handschuhs und des Roboters den Bereich jenseits der oben stehend erwähnten Kommunikationsöffnung erreicht.

4. Aseptisches System nach einem der Ansprüche 1 bis 3, worin das Inkubieren von Zellen im Inkubator vorgenommen wird.

5. Aseptisches System nach einem der Ansprüche 1 bis 4, worin der Isolator mit einem Zentrifugalabscheider (9) ausgestattet ist und eine dem menschlichen Körper oder einem Tier entnommene Körperflüssigkeit damit zentrifugiert wird.

6. Verfahren zur Verwendung eines aseptischen Systems (1), das einen Isolator (2), der von der äußeren Atmosphäre isoliert ist und in einem aseptischen Zustand im Inneren aufbewahrt wird, eine Oberflächensterilisierungsvorrichtung (5) zum Sterilisieren der Oberfläche eines Inkubationsbehältnisses und eine Erhitzungs- und Sterilisierungsvorrichtung (6) zum Erhitzen und Sterilisieren eines Inkubationsbehältnisses umfasst, worin, wenn das Inkubationsbehältnis aus dem Isolator (2) nach außen befördert wird, die Oberfläche des Inkubationsbehältnisses mittels der Oberflächensterilisierungsvorrichtung (5) sterilisiert wird, falls das Inkubieren im Inkubationsbehältnis normal vonstatten gegangen ist, und das Inkubationsbehältnis mittels der Erhitzungs- und Sterilisierungsvorrichtung (6) erhitzt und sterilisiert wird, falls das Inkubieren im Inkubationsbehältnis nicht normal vonstatten gegangen ist.

## Revendications

1. Système aseptique (1) comprenant un isolateur (2) qui est isolé de l'atmosphère extérieure et maintenu dans une condition aseptique à l'intérieur, des moyens de stérilisation de surface (5) pour stériliser la surface d'un objet à stériliser, les moyens de stérilisation de surface (5) ayant une porte (5B) pour faire entrer des matériaux provenant de l'extérieur du système (1) et pour faire sortir des matériaux à l'extérieur du système (1), le système aseptique comprenant en outre des moyens de chauffage et de stérilisation (6) pour chauffer et stériliser un objet à stériliser, les moyens de chauffage et de stérilisation (6) ayant une porte (6B) pour faire entrer les matériaux provenant de l'extérieur du système (1) et pour faire sortir les matériaux à l'extérieur du système (1),
**caractérisé en ce que** le système comprend en outre un incubateur (3) et dans lequel au moins trois des parois latérales de l'isolateur ont des ouvertures de communication par rapport aux moyens de stérilisation de surface (5), aux moyens de chauffage et de stérilisation (6) et à l'incubateur (3) respectivement, et chacune des ouvertures de communication est prévue avec une porte d'ouverture et de fermeture (5A, 6A, 3A) respectivement pour ouvrir et fermer les ouvertures de communication.

2. Système aseptique selon la revendication 1, dans lequel l'isolateur est formé selon une forme polygonale avec cinq parois latérales ou plus et au moins une paroi supplémentaire desdites cinq parois latérales ou plus a une ouverture de communication avec l'extérieur et l'ouverture de communication est prévue avec une porte d'ouverture et de fermeture (4A, 7A) pour ouvrir et fermer l'ouverture de communication.

3. Système aseptique selon la revendication 1 ou la revendication 2, dans lequel on prévoit au moins l'un parmi une demi-combinaison (8), un gant (216) et un robot (114) dans l'isolateur et la plage opérationnelle de la demi-combinaison, du gant et du robot atteint la plage située au-delà de l'ouverture de communication mentionnée ci-dessus.

4. Système aseptique selon l'une quelconque des revendications 1 à 3, dans lequel l'incubation des cellules est réalisée dans l'incubateur.

5. Système aseptique selon l'une quelconque des revendications 1 à 4, dans lequel l'isolateur est prévu avec un séparateur centrifuge (9) et un fluide corporel obtenu à partir d'un corps humain ou d'un animal est centrifugé avec ce dernier.

6. Procédé pour utiliser un système aseptique (1) comprenant un isolateur (2) qui est isolé de l'atmosphère externe et maintenu dans une condition aseptique à l'intérieur, des moyens de stérilisation de surface (5) pour stériliser la surface d'un récipient d'incubation et des moyens de chauffage et de stérilisation (6) pour chauffer et stériliser un récipient d'incubation, dans lequel lorsque le récipient d'incubation est retiré à l'extérieur de l'isolateur (2), dans le cas dans lequel l'incubation dans le récipient d'incubation s'est passée normalement, la surface du récipient d'incubation est stérilisée par les moyens de stérilisation de surface (5), et dans le cas dans lequel l'incubation dans le récipient d'incubation ne s'est pas passée normalement, le récipient d'incubation est chauffé et stérilisé par les moyens de chauffage et de stérilisation (6).
